# FASCICULE DE BREVET EUROPEEN

(11) **EP 0 700 870 B1**
(45) Date de publication et mention de la délivrance du brevet: **07.07.1999**
(21) Numéro de dépôt: 95402042.6
(22) Date de dépôt: 11.09.1995
(51) Int. Cl.: C01F 17/00, B01J 13/00

(54) **Dispersions colloidales d'un composé de cérium à pH élevé et leurs procédés de préparation**
Kolloidale Dispersionen einen Ceriumverbindung mit hoher pH, und Verfahren zu ihrer Herstellung
Colloidal dispersions of a cerium compound with high pH, and processes for their preparation

(30) Priorité: 12.09.1994 FR 9410856
(43) Date de publication de la demande: 13.03.1996
(73) Titulaire: RHODIA CHIMIE, 92408 Courbevoie Cédex (FR)
(72) Inventeur: Chane-Ching, Jean-Yves, F-95600 Eaubonne (FR); Chopin, Thierry, F-95320 Saint-Leu-La-Foret (FR); Persello, Jacques, F-25000 Besançon (FR)
(74) Mandataire: Dubruc, Philippe

(56) Documents cités:
- EP-A- 0 186 313
- EP-A- 0 206 906
- EP-A- 0 239 479
- EP-A- 0 316 205
- EP-A- 0 335 538
- FR-A- 2 652 805

## Description

La présente invention concerne des dispersions colloïdales d'un composé de cérium a pH élevé et leurs procédés de préparation.

Les dispersions de composés de cérium sont utilisées dans de nombreuses applications, on peut citer notamment l'utilisation en catalyse hétérogène et, en particulier en catalyse du traitement des gaz d'échappements des moteurs à explosion interne (catalyse pour post combustion automobile). Ces dispersions peuvent aussi être utilisées comme agent de revêtement anticorrosion ou en cosmétique.

De nombreux procédés de préparation de telles dispersions ont été décrits. Toutefois, les procédés connus ne permettent d'obtenir que des dispersions présentant un pH très acide, c'est à dire généralement inférieur à 5 et des taux d'impureté, c'est à dire une concentration en ions, élevés.

Or, dans les applications qui ont été mentionnées ci-dessus et notamment dans le cas de la cosmétique, il est important de pouvoir disposer de dispersions colloïdales ayant des pH moins acide et/ou des puretés plus élevées.

EP-A-316205 décrit des dispersions colloïdales d'oxyde de cérium dont le pH peut atteindre 5. Toutefois, ce document ne contient aucun enseignement sur des dispersions à pH supérieur à 5 et, en outre, les dispersions qui y sont mentionnées ne présentent pas une pureté élevée compte tenu de leur procédé de préparation.

L'objet de l'invention est donc de proposer des dispersions colloïdales à pH élevé et à grande pureté.

Dans ce but et selon un mode général de réalisation, la dispersion de l'invention est une dispersion en phase liquide aqueuse d'un composé de cérium à base d'oxyde ou d'hydroxyde de cérium IV, et elle est caractérisée en ce qu'elle présente un pH supérieur à 5 et une conductivité d'au plus 2mS/cm et en ce qu'elle a été obtenue par un procédé utilisant le nitrate de cérium comme produit de départ.

L'invention concerne aussi un procédé de préparation de la dispersion selon le mode général qui est caractérisé en ce qu'on part d'une première dispersion colloïdale, obtenue par un procédé utilisant le nitrate de cérium comme produit de départ, à pH inférieur au pH de la dispersion que l'on cherche à préparer et on élève le pH de cette première dispersion tout en diminuant sa force ionique et en ce qu'on réalise l'élévation du pH et la diminution de la force ionique en traitant la première dispersion par une résine cationique et par une résine anionique.

L'invention concerne aussi un procédé de préparation du type précité qui est caractérisé en ce qu'on ajoute à la dispersion de départ un acide organique.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, l'expression dispersion colloïdale d'un composé de cérium désigne tout système constitué de fines particules solides de dimensions colloïdales a base d'oxyde et/ou d'oxyde hydraté (hydroxyde) de cérium en suspension dans une phase liquide aqueuse, lesdites espèces pouvant en outre, éventuellement, contenir des quantités résiduelles d'ions liés ou adsorbés tels que par exemple des nitrates, des acétates, des citrates ou des ammoniums. On notera que dans de telles dispersions, le cérium peut se trouver soit totalement sous la forme de colloïdes, soit simultanément sous la forme d'ions et sous la forme de colloïdes.

En outre, on indique ici que le diamètre moyen des colloïdes doit être entendu comme désignant le diamètre hydrodynamique moyen de ces derniers, et tel que déterminé par diffusion quasi-élastique de la lumière selon la méthode décrite par Michael L. Mc CONNELL dans la revue Analytical Chemistry 53, n° 8, 1007 A, (1981).

Par ailleurs, le terme de pureté s'applique ici en référence aux impuretés sous forme d'espèces ioniques présentes dans la dispersion. Cette pureté peut s'exprimer en mesurant la conductivité de la dispersion.

Enfin, le composé de cérium est généralement un composé de cérium IV.

Dans le cas du mode général de réalisation de l'invention, la dispersion colloïdale présente à la fois un pH élevé qui supérieur à 5 et une grande pureté qui se traduit par une conductivité d'au plus 2mS/cm.

Selon un mode particulier de réalisation, la dispersion colloïdale présente les caractéristiques données ci-dessus et elle est caractérisée en outre en ce que les colloïdes présentent une teneur en carbone telle que le rapport moles de carbone/moles de cérium est d'au moins 0,01.

La dispersion colloïdale selon ce mode particulier de l'invention est en outre caractérisée par le fait que les colloïdes qui la constituent comprennent du carbone dans une proportion spécifique. Cette proportion est exprimée par le rapport moles de carbone/moles de cérium ou de CeO₂. Elle est d'au moins 0,01. Cette proportion est généralement d'au plus 0,5; elle peut être plus particulièrement comprise entre 0,1 et 0,15. Le carbone concerné ici correspond généralement à du carbone situé sur la surface des colloïdes.

Pour chacun des deux modes de réalisation décrits plus haut, le pH est préférentiellement d'au moins 6.

Pour chacun de ces modes existent deux variantes qui différent essentiellement par la taille des colloïdes.

Selon une première variante, le diamètre moyen des colloïdes de la dispersion peut varier entre 4 et 20nm et plus particulièrement entre 4 et 12nm.

Dans le cadre de cette première variante, la concentration de la dispersion colloïdale varie habituellement entre 10g/l et 250g/l et plus particulièrement entre 15g/l et 210g/l. Cette concentration est exprimée en CeO₂.

La conductivité varie en fonction du pH de la dispersion colloïdale. Elle est en principe d'autant plus faible que le pH est élevé. Cette conductivité, pour la première variante est d'au plus 2mS/cm. Elle peut être comprise entre 0,1 et 1,6mS/cm.

Le pH de la dispersion colloïdale est habituellement compris entre 5 et 7 et plus particulièrement entre 5 et 5,5.

Pour la seconde variante, le diamètre moyen des colloïdes est compris entre 40 et 100nm et plus particulièrement entre 40 et 60nm.

La concentration de la dispersion colloïdale varie habituellement entre 10g/l et 1000g/l et plus particulièrement entre 10g/l et 600g/l et encore plus particulièrement entre 15g/l et 300g/l.

La conductivité varie de la même manière qu'indiquée plus haut. Pour cette seconde variante, la conductivité est d'au plus 2mS/cm et de préférence d'au plus 1mS/cm. Plus particulièrement, elle peut être inférieure à 0,3mS/cm. Elle peut être même inférieure à 0,5mS/cm.

Le pH est compris habituellement entre 5 et 7 et plus particulièrement entre 5 et 6,5.

Une caractéristique préférentielle de la dispersion colloïdale de l'invention est que les colloïdes présentent une répartition monodisperse en taille.

Enfin, les dispersions de départ qui ont été obtenues par des procédés utilisant du nitrate de cérium comme produit de départ, présentent un rapport molaire NO₃/Ce qui est inférieur à 0,7 et peut être plus particulièrement compris entre 0,01 et 0,7 et encore plus particulièrement entre 0,15 et 0,3.

Les procédés de préparation des dispersions colloïdales de l'invention vont maintenant être décrits.

Ces procédés partent d'une première dispersion colloïdale dont le pH est inférieur à celui de la dispersion que l'on cherche à préparer.

La dispersion colloïdale de départ pourra avoir été obtenue par tout moyen connu. On pourra se référer notamment aux procédés décrits dans les demandes de brevet européens EP 206906, EP 208581, EP 316205. On peut utiliser tout particulièrement les dispersions colloïdales obtenues par thermohydrolyse d'une solution aqueuse d'un sel de cérium IV comme un nitrate, en milieu acide notamment. Un tel procédé est décrit dans la demande de brevet européen EP 239477 ou EP 208580. Pour obtenir les dispersions colloïdales monodisperses selon l'invention, on uilisera des dispersions de départ elles-mêmes monodisperses.

Quand on part notamment d'une suspension à concentration élevée en CeO₂ et qui peut donc présenter un taux d'impureté élevé et une tendance à décanter, il peut être utile de purifier par exemple par lavage/décantation la suspension ou la dispersion de départ. On laisse décanter la dispersion de départ après un ajout d'eau déionisée et on soutire le surnageant obtenu. On ajoute de nouveau de l'eau à la partie restante qui est remise en suspension. Cette opération peut être répétée plusieurs fois de suite jusqu'à l'obtention d'une conductivité donnée.

Le procédé pour la préparation des dispersions colloïdales selon les deux modes de réalisation consiste essentiellement à élever le pH de la dispersion de départ tout en diminuant sa force ionique.

Dans ce but, on utilise des résines échangeuses d'ions anioniques et cationiques.

On utilise de préférence des résines cationiques fortement acides et anioniques fortement basiques.

Les résines échangeuses d'ions cationiques et anioniques sont des produits bien connus. A titre d'exemple, on pourra mentionner pour les résines cationiques celles à squelette polystyrénique. On pourra utiliser plus particulièrement celles présentant des groupes fonctionnels sulfonates, H⁺. Des exemples de résines cationiques utilisables sont les résines Amberlite IR 120® ou Amberlite RN 77®.

A titre d'exemple, on pourra indiquer pour les résines anioniques celles à squelette de copolymères styrène-divinylbenzène. On pourra utiliser plus particulièrement celles présentant des groupes fonctionnels ammonium quaternaire, OH⁻. Des exemples de résines anioniques utilisables sont les résines Amberlite IRN 78® ou Duolite A 101®.

L'utilisation des deux types de résines est nécessaire. Cette utilisation peut être simultanée. Cette utilisation peut se faire aussi d'une manière alternée si la résine anionique, à laquelle seule est due la montée de pH, ne produit pas un incrément de pH trop important.

Le traitement par résine se fait de tout manière appropriée. Les résines peuvent être mises en contact direct avec la dispersion colloïdale. Une méthode préférée consiste à mettre les résines dans des sacs de membrane de dialyse du type décrit ci-dessus et ces sacs sont introduits dans la dispersion à traiter.

Le rapport de la masse de résine cationique à la masse de résine anionique peut être compris entre 0,1 et 1. La quantité de résine anionique à utiliser est définie par le pH que l'on souhaite atteindre. La cinétique relative au traitement par les résines peut être définie par le temps nécessaire pour obtenir un incrément de pH d'une unité. Ainsi, les résines peuvent être ajoutées de manière à obtenir un incrément d'une unité de pH entre 5 et 7000 minutes.

Le procédé de préparation de la dispersion colloïdale selon le mode particulier de réalisation de l'invention comporte une caractéristique supplémentaire.

Cette caractéristique consiste à ajouter à la première dispersion de départ un acide organique.

Cet acide organique est de préférence choisi dans le groupe des acides solubles dans l'eau et présentant un pKa compris entre 2,5 et 5,0. Ce peut être notamment un acide aliphatique et plus particulièrement un acide aliphatique saturé. On peut citer à titre d'exemple les acides formique, acétique, propionique, butyrique, valérique, citrique et malonique.

La quantité d'acide ajoutée est habituellement comprise entre 0,1 et 20% en moles d'acide par rapport à CeO₂.

La suite du procédé est identique à ce qui a été décrit pour la préparation des dispersions colloïdales selon le mode général de réalisation, c'est à dire que simultanément ou postérieurement à l'addition d'acide, on diminue la force ionique de la dispersion.

Il est possible d'effectuer une étape supplémentaire ultérieure pour obtenir des dispersions colloïdales plus concentrées.

Cette concentration peut être réalisée par évaporation, par compression osmotique ou par ultrafiltration.

La compression osmotique est la méthode dont le principe consiste à équilibrer le potentiel chimique de l'eau à travers une membrane.

On procède en disposant la dispersion colloïdale dans un sac à dialyse par exemple en matière cellulosique, ce sac étant placé dans une solution aqueuse dont le potentiel chimique de l'eau est différent de celui de la phase aqueuse de la dispersion. Ceci peut se faire par exemple en utilisant une solution aqueuse de polyéthylène glycol (PEG) et de NaNO₃. La concentration en PEG fixe la pression osmotique et donc la concentration finale de la dispersion colloïdale de composé de cérium.

L'ultrafiltration peut se faire en alternant des séquences d'ultrafiltration et de dilution de la suspension ultrafiltrée par une solution d'eau déionisée.

L'ensemble des étapes décrites plus haut des procédés est réalisé de préférence à température ambiante.

Les dispersions colloïdales de l'invention telles que décrites plus haut ou telles qu'obtenues par les procédés qui viennent d'être détaillés peuvent être utilisées dans de nombreuses applications. On peut citer la catalyse pour post combustion automobile, la lubrification, les céramiques. Elles peuvent plus particulièrement être utilisées dans des compositions cosmétiques notamment dans la préparation de crèmes anti-UV. Elles peuvent être utilisées aussi sur un substrat en tant qu'agent anticorrosion.

Ainsi, les dispersions colloïdales de l'invention s'appliquent tout particulièrement au traitement de substrats métalliques et notamment aux substrats en acier contenant du chrome et/ou de l'aluminium ou aux substrats en alliage contenant du chrome et/ou de l'aluminium.

On peut citer ici à titre d'exemple les aciers inoxydables du type martensitiques, ferriques, austénitiques, ces derniers pouvant être stabilisés au titane ou au niobium. On peut mentionner aussi les aciers ou les alliages réfractaires comme les alliages Fe-Cr-Al, Ni-Cr-Al-Y, Co-Cr-Al-Y ou encore Fe-Al ou Ni-Al.

L'état du substrat avant le traitement ne nécessite pas d'intervention particulière, si ce n'est les traitements classiques de dégraissage et de nettoyage. Les substrats peuvent être ou non pré-oxydés.

Le dépôt de la dispersion sur le substrat peut être réalisé directement à partir de la suspension de l'invention en utilisant des techniques classiques de revêtement du type trempage ou pulvérisation par exemple.

Après réalisation du dépôt, le substrat présente une couche adhérente en surface, ce qui le rend manipulable.

Le substrat doit être ensuite traité thermiquement afin d'éliminer l'eau notamment.

Le traitement thermique se fait généralement à une température d'au plus 600°C. Cette température peut être plus basse, par exemple d'au plus 400°C, en fonction de la nature des substrats.

Des exemples vont maintenant être donnés pour illustrer l'invention ou au moins certains de ses aspects. Dans ces exemples, la conductivité est mesurée à l'aide d'un conductimètre de type CDM 83 (Radiometer Copenhaguen) et de sa cellule de mesure de type CDC 304.

### EXEMPLE 1

Dans un flacon en pyrex, on verse 200 cc de solution colloïdale aqueuse, obtenue par addition d'eau à un composé de cérium ⁴⁺ dispersable, synthétisé comme décrit dans le brevet EP 208580

Le diamètre des colloïdes est de 5 nm, la concentration en cérium ⁴⁺ exprimée en CeO₂ est de 172 g/l. Sa conductivité est de 57 mS/cm et son pH égal à 1,1. Un sac à dialyse en matière cellulosique (diamètre de coupure de PM 13000 à 15000) fermé et contenant 18 g de résine cationique RH (Amberlite IRN 77), simultanément à un sac à dialyse et contenant 20 g de résine anionique ROH (Amberlite IRN 78) sont immergés dans la solution colloïdale. La solution colloïdale est ainsi maintenue en présence des résines à température ambiante dans le flacon en pyrex bouché.

Les conditions de renouvellement des résines ainsi que les évolutions de pH et conductivité de la solution colloïdale sont décrites dans le tableau suivant :

**Tableau 1**

| **Date** | **pH avant introduction des résines** | **Conductivité avant introduction des résines** | **Masse de résine RH introduite** | **Masse de résine ROH introduite** |
|---|---|---|---|---|
| 0 | 1,1 | 57 mS/cm | 18 g | 20 g |
| 3 jours | 1,32 | 34,6 mS/cm | 17 g | 18 g |
| 6 jours | 1,68 | 16,9 mS/cm | 18 g | 17,5 g |
| 8 jours | 2,69 | 2,14 mS/cm | 17 g | 17 g |
| 10 jours | 3,3 | 1,82 mS/cm | 17 g | 17 g |
| 13 jours | 3,93 | 1,62 mS/cm | 19 g | 18 g |
| 15 jours | 3,97 | 1,58 mS/cm | 17,5 g | 17,5 g |
| 17 jours | 4,04 | 1,57 mS/cm | 17,5 g | 18 g |
| 20 jours | | | 16,5 g | 16,5 g |
| 22 jours | 4,54 | | 17,5 g | 18,2 g |
| 24 jours | 5,4 | 1,54 mS/cm | 0 | 0 |

La concentration en CeO₂ déterminée par séchage à l'étuve d'une aliquote de solution colloïdale et calcination à 1000°C est égale à 138 g/l. La solution colloïdale obtenue est stable au moins 6 mois vis à vis de la décantation et de la gélification. Les colloïdes présentent des diamètres moyens de 5 nm.

### EXEMPLE 2

A 500 grammes de composé de cérium ⁴⁺, dispersable, obtenu par thermohydrolyse de solutions de nitrate cérique (CeO₂ = 60 g/l et r = OH/Ce⁴⁺ = 2) comme décrit dans le brevet EP 208 580 sont additionnés 400 cc d'eau déionisée.

Une première phase de purification de la dispersion est effectuée par décantation et soutirage du surnageant. Après homogénéisation de la dispersion précédemment obtenue par agitation, on laisse le précipité décanter une nuit et on soutire le surnageant. On additionne de nouveau 300 cc d'eau déionisée et on homogénéise la dispersion par agitation. On laisse de nouveau décanter une nuit et on soutire le surnageant. On additionne 150 cc d'eau déionisée et après homogénéisation, la majeure partie du composé de cérium ⁴⁺ est sous forme colloïdale. Après élimination de la faible partie non dispersée, on recueille une solution colloïdale présentant une concentration en CeO₂ d'environ 510 g/l en CeO₂.

On verse 1 litre de cette solution colloïdale ainsi préparée à 172 g/l en CeO₂ dans un flacon en pyrex. Le diamètre des colloïdes est de 5 nm. Le pH est égal à 1,56 et la conductivité est de 21,1 mS/cm.

Quatre sacs à dialyse, en matière cellulosique (diamètre de coupure PM 13000 à 15000) fermés et contenant chacun environ 15 g de résine cationique RH (Amberlite IRN 77), simultanément à quatre sacs à dialyse identiques et contenant chacun environ 15 g de résine anionique ROH (Amberlite IRN 78) sont immergés simultanément dans la solution colloïdale. La solution colloïdale est ainsi maintenue en présence de résines à température ambiante dans le flacon en pyrex bouché.

Les conditions de renouvellement des résines, ainsi que les évolutions de pH et de la conductivité de la solution colloïdale sont décrites dans le tableau suivant :

**Tableau 2**

| **Date** | **pH avant introduction des résines** | **Conductivité avant introduction des résines** | **Masse de résine RH introduite** | **Masse de résine ROH introduite** |
|---|---|---|---|---|
| 0 | 1,56 | 21,1 mS/cm | 4 x 15 g | 4 x 15 g |
| 2 jours | 1,76 | 9,7 mS/cm | 4 x 15 g | 4 x 15 g |
| 8 jours | 2,8 | 2,3 mS/cm | 4 x 15 g | 4 x 15 g |
| 10 jours | 3,7 | 1,7 mS/cm | 4 x 15 g | 4 x 15 g |
| 13 jours | 3,86 | 1,64 mS/cm | 4 x 15 g | 4 x 15 g |
| 15 jours | 39 | | 4 x 15 g | 4 x 15 g |
| 17 jours | 4,11 | 1,62 mS/cm | 4 x 16 g | 4 x 15 g |
| 20 jours | 4,22 | 1,5 mS/cm | 4 x 15 g | 4 x 15 g |
| 22 jours | 4,61 | 1,38 mS/cm | 0 | 0 |

Une concentration de la solution colloïdale est effectuée par compression osmotique comme suit.

La solution colloïdale est versée dans la membrane cellulosique décrite précédemment et fermée à son extrémité inférieure.

L'ensemble est équilibré par une solution de NaNO₃ 0,005 M et à pH de 4,61 pendant 7 jours.

L'ensemble membrane cellulosique/solution colloïdale est alors immergé dans une solution aqueuse de composition suivante :
- 5% en poids de polyéthylène glycol PEG de poids moléculaire 35000
- 5.10⁻³ NaNO₃ et
- pH = 4,61 (HNO₃).

On recueille la solution colloïdale au bout de 3 jours.

La concentration de la solution colloïdale est déterminée égale a 178 g/1000 g de dispersion, ce qui est équivalent à 210 g/l en CeO₂.

La taille moyenne des colloïdes est de 5 nm.

### EXEMPLE 3

A 500 cc de solution colloïdale, de colloïdes de diamètre 50 nm, concentrée à 705 g/l en CeO₂ en utilisant la même technique qu'a l'exemple 2, de pH 1,04 et de conductivité 43,9 mS/cm, trois sacs à dialyse, en matière cellulosique (diamètre de coupure 13000 à 15000) fermés et contenant chacun environ 20 g de résine cationique RH (Amberlite IRN 77), simultanément à trois sacs à dialyse identiques et contenant chacun environ 20 g de résine anionique ROH (Amberlite IRN 78) sont immergés dans la solution colloïdale. La solution colloïdale est ainsi maintenue en présence des résines à température ambiante dans le flacon en pyrex bouché.

Les conditions de renouvellement des résines, ainsi que les évolutions de pH et de conductivité de la solution colloïdale sont décrites dans le tableau suivant :

**Tableau 3**

| **Date** | **pH avant introduction des résines** | **Conductivité avant introduction des résines** | **Masse de résine RH introduite** | **Masse de résine ROH introduite** |
|---|---|---|---|---|
| 0 | 104 | 43,9 mS/cm | 3 x 20 g | 3 x 20 g |
| 5 jours | 1,2 | 19,8 mS/cm | 3 x 20 g | 3 x 20 g |
| 7 jours | 1,9 | 6,11 mS/cm | 3 x 20 g | 3 x 20 g |
| 10 jours | 2,46 | 2,56 mS/cm | 3 x 20 g | 3 x 20 g |
| 12 jours | 2,54 | 2,14 mS/cm | 3 x 20 g | 3 x 20 g |
| 14 jours | 2,86 | 1,68 mS/cm | 3 x 20 g | 3 x 20 g |
| 17 jours | 3,07 | 1,56 mS/cm | 3 x 20 g | 3 x 20 g |
| 19 jours | 3,24 | 1,24 mS/cm | 3 x 30 g | 3 x 30 g |
| 21 jours | 3,40 | 1,19 mS/cm | 3 x 30 g | 3 x 30 g |
| 25 jours | 3,51 | 1,00 mS/cm | 3 x 30 g | 3 x 30 g |
| 31 jours | 3,52 | 0,92 mS/cm | 3 x 30 g | 3 x 30 g |
| 35 jours | 3,75 | 0,84 mS/cm | 3 x 30 g | 3 x 30 g |
| 38 jours | 4,01 | 0,80 mS/cm | 0 | 0 |

Une concentration de la solution colloïdale est effectuée par compression osmotique comme suit :

La solution colloïdale est versée dans la membrane cellulosique décrite précédemment et fermée à son extrémité inférieure.

L'ensemble est équilibré dans une solution de NaNO₃ 0,0075 M et à un pH de 4 pendant 7 jours.

L'ensemble membrane/solution colloïdale est alors immergé dans une solution aqueuse de composition suivante :
- 5% PEG, PM 35000
- NaNO₃ 0,0075 M
- pH 4

On recueille la solution colloïdale au bout de 2 jours.

La concentration de la solution colloïdale déterminée par séchage et calcination d'une aliquote de solution colloïdale est de 518 g de CeO₂/1000 g de dispersion, soit une concentration équivalente de 935 g/l en CeO₂.

Le diamètre moyen des colloïdes est de 50 nm.

### EXEMPLE 4

200 cc d'une solution colloïdale de CeO₂ à 81 g/l en CeO₂ sont introduits dans un bêcher. Les colloïdes présentent un diamètre moyen de 50 nm. Le pH de la solution colloïdale est de 1. La conductivité ionique est de 32 mS/cm. On additionne sous agitation 1,2 g d'acide acétique concentré commercial (PROLABO), soit un rapport molaire acétique/CeO₂ d'environ 20%, 20 cc d'une résine cationique (Amberlite IRN 77) préalablement traitée à l'aide d'une solution HCL 1N et lavée à l'eau désionisée jusqu'à pH 6, sont incorporés à la solution colloïdale sous agitation. Au bout de 12 mn, on introduit 10 cc de résine anionique (Amberlite 78) traitée par NaOH 1M et lavée à l'eau jusqu'à pH 6. Ce cycle est répété jusqu'à obtenir un pH de 6,5. Après séparation de la résine par décantation, la solution colloïdale présente une conductivité ionique de 48 µS/cm. Les colloïdes présentent une taille de 50 mn. La solution est parfaitement stable au cours du temps. La teneur en nitrate exprimée en NO₃/CeO₂ est de 0,66% en poids. Le dosage en carbone indique un pourcentage de 0,97% de C/CeO₂. La concentration en CeO₂ est de 60 g/l.

### EXEMPLE 5

On répète l'exemple 4 en remplaçant l'acide acétique par de l'acide citrique. On introduit 1,2 g d'acide citrique solide à 1H20, préalablement dissous dans 10 cc d'eau dans la solution colloïdale sous agitation. Le rapport citrique/CeO₂ est de 2% molaire.

L'addition de résine cationique/anionique est effectuée comme à l'exemple 4 jusqu'à pH 6. Après séparation de la résine par décantation, la solution colloïdale présente une concentration en CeO₂ de l'ordre de 60 g/l. La solution est parfaitement stable au cours du temps.

### EXEMPLE 6

200 cc d'une solution colloïdale de CeO₂ à 141 g/l en CeO₂ sont introduits dans un bêcher de 1 litre. On additionne sous agitation 100 cc d'eau. Le pH est de 1,1 et la conductivité de 29,9 mS/cm. L'examen à la diffusion quasi-élastique de la lumière montre des particules d'environ 5 nm.

On additionne 50 cc de résine cationique (Amberlite IR 120) préalablement traitée à l'acide chlorhydrique. A t = 8 mn, on additionne de nouveau 50 cc de résine cationique. A t = 16 mn, addition supplémentaire de 50 cc de résine cationique. A t = 24 mn, addition supplémentaire de 50 cc de résine cationique. Après arrêt de l'agitation à t = 32 mn, on élimine les résines du milieu réactionnel par décantation. La solution colloïdale ainsi recueillie est alors traitée suivant le cycle : 25 cc de résine anionique (Duolite A 101, traitée NaOH) et 8 mn après 25 cc de résine cationique. Ce cycle est répété jusqu'à obtention de pH 7.

Après arrêt de l'agitation et décantation, on élimine les résines. La solution colloïdale ainsi recueillie présente une concentration de 30 g/l en CeO₂. L'examen à la diffusion quasi-élastique de la lumière montre des colloïdes de diamètre moyen de 50 nm indiquant une agglomération des colloïdes primaires. La conductivité ionique est de 152 µS/cm. La solution est stable vis à vis de la décantation et de la gélification au cours du temps.

Sur une aliquote, on ultracentrifuge et on récupère les colloïdes par décantation. Le dosage en NO₃ sur les colloïdes solides montre une teneur en NO₃/CeO₂ de l'ordre de 0,3% en poids.

## Revendications

1. Dispersion colloïdale en phase liquide aqueuse d'un composé de cérium à base d'oxyde ou d'hydroxyde de cérium IV, caractérisée en ce qu'elle présente un pH supérieur à 5 et une conductivité d'au plus 2mS/cm et en ce qu'elle a été obtenue par un procédé utilisant le nitrate de cérium comme produit de départ.

2. Dispersion selon la revendication 1, caractérisée en ce qu'elle présente un pH d'au moins 6.

3. Dispersion selon l'une des revendications précédentes, caractérisée en ce que le diamètre moyen de ses colloïdes est compris entre 4 et 20nm.

4. Dispersion selon l'une des revendications précédentes, caractérisée en ce que le diamètre moyen de ses colloïdes est compris entre 40 et 100nm.

5. Dispersion selon la revendication 4, caractérisée en ce qu'elle présente une conductivité d'au plus 1mS/cm.

6. Dispersion selon la revendication 4 ou 5, caractérisée en ce qu'elle présente une conductivité d'au plus 0,3mS/cm.

7. Procédé de préparation d'une dispersion selon l'une des revendications précédentes caractérisé en ce qu'on part d'une première dispersion colloïdale, obtenue par un procédé utilisant le nitrate de cérium comme produit de départ, à pH inférieur au pH de la dispersion que l'on cherche à préparer et on élève le pH de cette première dispersion tout en diminuant sa force ionique et en ce qu'on réalise l'élévation du pH et la diminution de la force ionique en traitant la première dispersion par une résine cationique et par une résine anionique.

8. Procédé selon la revendication 7, caractérisé en ce qu'on ajoute à la dispersion de départ un acide organique.

9. Procédé selon la revendication 8, caractérisé en ce qu'on utilise comme acide organique un acide choisi dans le groupe des acides solubles dans l'eau et présentant un pKa compris entre 2,5 et 5,0.

10. Procédé selon l'une des revendications 7 à 9, caractérisé en ce qu'il comprend une étape supplémentaire ultérieure de concentration de la dispersion.

11. Procédé selon la revendication 10, caractérisé en ce qu'on effectue la concentration de la dispersion pu évaporation, par compression osmotique ou par ultrafiltration.

12. Utilisation sur un substrat comme agent anticorrosion d'une dispersion selon l'une des revendications 1 à 6 ou d'une dispersion obtenue par le procédé selon l'une des revendications 7 à 11.

13. Utilisation dans une composition cosmétique d'une dispersion selon l'une des revendications 1 à 6 ou d'une dispersion obtenue par le procédé selon l'une des revendications 7 à 11.

14. Utilisation en catalyse pour post combustion automobile, en lubrification ou dans les céramiques d'une dispersion selon l'une des revendications 1 à 6 ou d'une dispersion obtenue par le procédé selon l'une des revendications 7 à 11.

## Claims

1. A colloidal dispersion, in an aqueous liquid phase, of a cerium compound based on cerium IV oxide or hydroxide, characterized in that its pH is over 5 and its conductivity is at most 2 mS/cm and in that it has been obtained by a process using cerium nitrate as starting product.

2. A dispersion according to claim 1, characterized in that its pH is at least 6.

3. A dispersion according to any one of the preceding claims, characterized in that the mean diameter of said colloids is in the range 4 to 20 nm.

4. A dispersion according to any one of the preceding claims, characterized in that the mean diameter of said colloids is in the range 40 to 100 nm.

5. A dispersion according to claim 4, characterized in that its conductivity is at most 1 mS/cm.

6. A dispersion according to claim 4 or claim 5, characterized in that its conductivity is at most 0.3 mS/cm.

7. A process for preparing a dispersion according to any one of the preceding claim characterized in that, starting form a first colloidal dispersion obtained by a process using cerium nitrate as starting product, with a pH lower than the pH of the dispersion to be prepared, and raising the pH of said first dispersion while reducing its ionic strength, and in that the pH is raised and the ionic strength is reduced by treating said first dispersion with a cationic resin and with an anionic resin.

8. A process according to claim 7, characterized in that an organic acid is added to the starting dispersion.

9. A process according to claim 8, characterized in that the organic acid is an acid selected from the group formed by water-soluble acids with a pKa in the range 2.5 to 5.0.

10. A process according to any one of claims 7 to 9, characterized in that it comprises a subsequent supplementary step of concentrating the dispersion.

11. A process according to claim 10, characterized in that the dispersion is concentrated by evaporation, by osmotic compression or by ultrafiltration.

12. Use of a dispersion according to any one of claims 1 to 6 or of a dispersion obtained by the process according to any one of claims 7 to 11, on a substrate as an anti-corrosion agent.

13. Use of a dispersion according to any one of claims 1 to 6 or of a dispersion obtained by the process according to any one of claims 7 to 11, in a cosmetic composition.

14. Use of a dispersion according to any one of claims 1 to 6 or of a dispersion obtained by the process according to any one of claims 7 to 11, in post-combustion automotive catalytic converters, for lubrication or in ceramics.

## Patentansprüche

1. Kolloidale Dispersion in flüssiger wässeriger Phase bestehend aus einer Cer-Verbindung auf der Grundlage eines Cer(IV)-oxids oder-hydroxids, dadurch gekennzeichnet, daß diese einen pH-Wert von mehr als 5 und eine spezifische elektrische Leitfähigkeit von höchstens 2 ms/cm aufweist und dadurch daß diese durch ein Cerium Nitrat als Ausganprodukt benutzendes Verfahren hergestellt worden ist.

2. Dispersion nach Anspruch 1, dadurch gekennzeichnet, daß sie einen pH-Wert von mindestens 6 aufweist.

3. Dispersion nach einem der vorgenannten Ansprüche, dadurch gekennzeichnet, daß der mittlere Durchmesser ihrer Kolloide zwischen 4 und 20 nm liegt.

4. Dispersion nach einem der vorgenannten Ansprüche, dadurch gekennzeichnet, daß der mittlere Durchmesser ihrer Kolloide zwischen 40 und 100 nm liegt.

5. Dispersion nach Anspruch 6, dadurch gekennzeichnet, daß die spezifische elektrische Leifähigkeit höchstens 1 mS/cm beträgt.

6. Dispersion nach einem der Ansprüche 4 oder 5, dadurch gekennzeichnet, daß die spezifische elektrische Leitffähigkeit höchstens 0,3 mS/cm beträgt.

7. Verfahren zur Herstellung einer Dispersion nach einem der vorgenannten Ansprüche dadurch gekennzeichnet, daß von einer durch ein Cerium Nitrat als Ausgangprodukt benutzendes Verhfaren hergestellten ersten kolloidalen Dispersion ausgegangen wird, die einen pH-Wert aufweist, der niedriger als der pH-Wert der herzustellenden Dispersion ist, daß der pH-Wert dieser ersten Dispersion erhöht wird, indem gleichzeitig ihre Ionenstärke verringert wird, und daß die Erhöhung des pH-Wertes und die Verringerung der Ionenstärke erreicht wird, indem die erste Dispersion mit einem Kationenaustauscherharz und einem Anionenaustauscherharz behandelt wird.

8. Verfahren nach Anspruch 7 dadurch gekennzeichnet, daß eine organische Säure zu der Dispersion gegeben wird.

9. Verfahren nach Anspruch 8, dadurch gekennzeichnet, daß als Organische Säure eine aus der Gruppe der Wasserlöslichen Säuren mit einem pKs zwischen 2,5 und 5 ausgewählte Säure verwendet wird.

10. Verfahren nach einem der Ansprüche 7 bis 9, dadurch gekennzeichnet, daß sich ein zusätzlicher Verfahrensschritt zur Konzentrierung der Dispersion anschließt.

11. Verfahren nach Anspruch 10, dadurch gekennzeichnet, daß die Konzentrierung der Dispersion durch Verdunstung, osmotischen Druck oder Utrafiltration erreicht wird.

12. Verwendung einer Dispersion nach einem der Ansprüche 1 bis 6 oder einer Dispersion hergestellt durch ein Verfahren nach einem der Ansprüche 7 bis 11auf einem Substrat als Korrosionsschutzmittel.

13. Verwendung einer Dispersion nach einem der Ansprüche 1 bis 6 oder einer Dispersion hergestellt durch ein Verfahren nach einem der Ansprüche 7 bis 11 in einer kosmetisch Zusammensetzung.

14. Verwendung einer Dispersion nach einem der Ansprüche 1 bis 6 oder einer Dispersion hergestellt durch ein Verfahren nach einem der Ansprüche 7 bis 11 bei der Katalyse zur Nachverbrennung in Fahrzeugen, bei der Schmierung oder in Keramiken.
